(19) Europäisches Patentamt
European Patent Office
Office européen des brevets

(11) **EP 3 441 078 A1**

(12) **EUROPEAN PATENT APPLICATION**
published in accordance with Art. 153(4) EPC

(43) Date of publication:
**13.02.2019 Bulletin 2019/07**

(21) Application number: **16897544.9**

(22) Date of filing: **07.04.2016**

(51) Int Cl.:
*A61K 36/8984* (2006.01)      *A61K 36/52* (2006.01)
*A61K 36/282* (2006.01)      *A61K 36/258* (2006.01)
*A61K 9/48* (2006.01)      *A61P 37/02* (2006.01)

(86) International application number:
**PCT/CN2016/078649**

(87) International publication number:
**WO 2017/173609 (12.10.2017 Gazette 2017/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Beijing Lu De Kai Qi Co., Ltd.**
**Beijing 100000 (CN)**

• **Lu, Wei**
**Beijing 100000 (CN)**

(72) Inventor: **LU, Wei**
**Beijing 100000 (CN)**

(74) Representative: **Dr. Gassner & Partner mbB**
**Wetterkreuz 3**
**91058 Erlangen (DE)**

(54) **PHARMACEUTICAL PRODUCT FOR TREATING LUPUS ERYTHEMATOSUS AND COMBINED IMMUNE DEFECT, AND PREPARATION AND APPLICATION THEREOF**

(57)      An anti-lupus erythematosus pharmaceutical product for enhancing immunity is prepared from the following raw materials (in parts by weight): walnut extract (1), wormwood extract (0.5-2), American ginseng extract (1-5), and shí hú extract (1-10). The pharmaceutical product is prepared by supercritical fluid extraction, spray drying and the like. The pharmaceutical product can enhance immunity, and prevent or treat lupus erythematosus.

**EP 3 441 078 A1**

**Description**

**Technical Field**

The present invention relates to the field of medicine, and more particularly, to an anti-lupus erythematosus pharmaceutical product for combined immunization, and a preparation method and use thereof.

**Background Art**

Lupus erythematosus (LE) is an autoimmune connective tissue disease. Lupus erythematosus is classified into discoid lupus erythematosus (DLE), subacute cutaneous lupus erythematosus (SCLE), systemic lupus erythematosus (SLE), lupus erythematosus panniculitis (LEP), neonatal lupus erythematosus (NLE), drug-induced lupus erythematosus (DIL) and other subtypes. At present, the cause of lupus erythematosus is not fully understood, but the medical community generally believes that the cause of lupus erythematosus may be related to heredity, sexual hormones, environment and other factors that cause immune function disorders. In the prior art, the treatment of lupus erythematosus mainly includes western medicine therapies and Chinese medicine therapies. Western medicine has a certain effect on the treatment of lupus erythematosus, and especially in the outbreak period or acute active period of the disease, using large doses of hormones and cyclophosphamide pharmaceutical products has a positive effect on the rapid control of the disease. However, long-term use of these pharmaceutical products in large quantities can cause side effects, damage to the body, and even be life-threatening, and lupus erythematosus is prone to recurrent attacks. Chinese medicine has a long treatment period for lupus erythematosus, and various tissues and organs such as skin and internal organs are damaged extensively, which affects the confidence of patients to cure, resulting in a general therapeutic effect.

Song Xiaoping et al. proposed Chinese medicine and Western medicine therapies of cutaneous lupus erythematosus in Progress and Thinking on the Treatment of Cutaneous Lupus Erythematosus with Chinese Medicine and Western Medicine ("Hainan Medicine", 2010, Vol. 21, No. 21). However, the compatibility of western medicine and Chinese medicine is still to be studied, and the combination of Chinese and Western medicine therapies is mainly to strengthen the patient's immunity and combine western medicine to achieve the purpose of treatment. This method can only enhance the immunity of patients during treatment, and cannot repair the immune system that was destroyed before treatment or cannot ensure the balance of immunity in the body environment, which will lead to easy recurrence of the disease; and the two therapies easily interfere with each other and are prone to side reactions.

How to treat lupus erythematosus quickly, effectively and safely and prevent it from recurring has become one of the key research directions in the industry.

**Summary of the Invention**

In view of the deficiencies of the prior art, the present invention is directed to a anti-lupus erythematosus pharmaceutical product for combined immunization, which is capable of inhibiting and killing bacteria, enhancing the body immunity and quickly and effectively treating lupus erythematosus, and is also capable of further enhancing the body immunity, adjusting the in-vivo environment of the body, restoring normal balance of the body and preventing lupus erythematosus from recurring.

In order to achieve the above object, the present invention provides the following technical solutions: An anti-lupus erythematosus pharmaceutical product for combined immunization comprises an extract of a walnut, an extract of a wormwood, an extract of an American ginseng and an extract of shí hú, the extract of a walnut, the extract of a wormwood, the extract of an American ginseng and the extract of a shí hú are in a weight ratio of 1:(0.5-2):(1-5):(1-10).

Preferably, the walnut is black walnut, the wormwood is *Artemisia absinthium,* the American ginseng is semi-wild American ginseng, and the shí hu is wild shí hú.

Walnut is *Juglans regia L.* belonging to *Juglandaceae.* The walnut extract according to the present invention is prepared by extracting the endothelium of Black Walnut (*Juglans nigra L*), which contains juglone, flavones, tannin, polysaccharide, gallic acid, volatile oil, mucilaginous gum, albumin, mineral, cellulose, linoleic acid, glyceride, linolenic acid, glyceryl oleate, riboflavin, carotene, gemin D, casurin, and *Rosa davurica Pall* extract. It has been found that the active ingredients in different ratios can achieve the object of the present invention, and the walnut extract prepared by the preparation method according to the present invention is most effective.

Wormwood, also known as folium artemisiae argyi, felon herb, Chinese mugwort or Jia Ai, is a dry leave of *Artemisia argyi Levi.et Vant.* belonging to *Compositae.* It is pungent, bitter and warm, has slight toxicity and can dispel coldness to relieve pain and warming meridian to stop bleeding. The wormwood extract according to the present invention is preferably prepared by extracting the whole herb of *Artemisia absinthium* of North America (wormwood or *Artemisia absinthium*). The main component of this extract is volatile oil, and the oil contains terpinenol-4, $\beta$-caryophyllene, artemisia alcohol, linalool, camphorae, borneol, cineol (eucalyptol), trans-carveol, $\alpha$-terpinenol, etc., and polysaccharides, tannic acid, thujone, phellandrene, β-sitosterol, 5,7-dihydroxy-6,3',4'-trimethyl flavonee. A study has found that the difference in ratios of active ingredients can achieve the object of the present invention, and the wormwood extract prepared by the preparation method according to the present invention is most effective.

American ginseng, also known as Huaqi ginseng, Yang ginseng, American ginseng, is the root of *Panax quinquefolium Linn* belonging to Araliaceae, Panax, and belongs to the same family and the same genus as Panax ginseng abounding in northeastern China. It is cold in nature, and has the functions of nourishing YIN, tonifying QI, reducing blood pressure and relieving heat. Semi-wild American ginseng is one of the American ginseng varieties. Its main producing areas are 25 states including Wisconsin, New York, and Kentucky. The semi-wild American ginseng is cultivated by placing the seeds of American ginseng in a wild-like environment. The American ginseng extract of the present invention is extracted from the body of the semi-wild American ginseng. Its main ingredients are ginsenosides, volatile oil, fatty acids in grease, organic acids, amino acids, trace elements, carbohydrates, pectin and sterol. The ginsenosides mainly include ginsenosides Ro, $Rb_1$, $Rb_2$, $Rb_3$, Rc, Rd, Re, Rf, $Rg_1$, $Rg_2$, $Rg_3$, $Rh_1$ and $R_{AO}$, American ginseng saponin R1, gypenoside XI, gypenoside XVII. Pseudoginsenoside mainly include $F_{11}$, ginsenosides $F_2$ and $F_3$, etc. The volatile oil mainly includes β-farnesene, sesquiterpenoids, bisabolene, α-curcumene, etc. The study finds that different ratios of active ingredients can achieve the object of the present invention, and the American ginseng extract prepared by the preparation method of the present invention has the best effect.

Shí hú, also called Diaolan, Dulan, is the stem of Dendrobium nobile Lindl, Dendrobium candidum Wall.ex Lindl. or Dendrobium fimbriatum hook. Var. oculatum Hook and its allied species. It is sweet and slightly cold, and is capable of reinforcing the stomach, promoting salivation, nourishing YIN and clearing heat. The shí hú extract of the present invention is extracted from the stem of wild Dendrobium nobile Lindl, and contains a variety of alkaloids, polysaccharides, amino acids, mucilages, starchs, phenanthrenes and bibenzyls. The alkaloids include dendrobine, dendrine, dendramine, dendroxine, 6-hydroxy-dendroxine, etc. The study finds that different ratios of active ingredients can achieve the object of the present invention, and the shí hu extract prepared by the preparation method of the present invention has the best effect.

The study finds that in the anti-lupus erythematosus pharmaceutical product for combined immunization of the present invention, the weight ratio of the walnut extract to the wormwood extract to the American ginseng extract to the shí hú extract is 1:(0.5-2):(1-5):(1-10), and the combination has the following synergistic effects:

(1) for patients with lupus erythematosus, it can relieve and improve symptoms, for example, relieve pain, eliminate itching, increase appetite, ease pain, tonify the blood, regenerate blood, protect the liver, and keep the blood sugar stable;

(2) it can inhibit and kill bacteria, inhibit viruses, expel toxins and resist inflammation, has strong oxidation resistance and hydroxyl radical-scavenging ability, and enhances the ability of patients with lupus erythematosus to fight other new diseases;

(3) other ingredients combined with its rich polysaccharides have the effect of enhancing the body immunity, can promote the humoral immune function and cellular immune function of the body and induce the generation of a variety of cytokines, and can quickly and effectively treat lupus erythematosus;

(4) it promotes blood activity, regulates the central nervous system, protects the cardiovascular system, enhances the toxin-expelling function of the body, enhances the immunity and energy of the body, provides the nutrients required for the body, enhances the cellular immune activity, regulates the in vivo environment of the body and repair the original damaged immune function, restores the normal physiological balance and prevents lupus erythematosus from recurring.

The study finds that preferably, the weight ratio of the walnut extract to the wormwood extract to the American ginseng extract to the shí hú extract is 1:1:5:10.

Preferably, a formulation of the pharmaceutical product is a capsule.

Administration in the form of capsule is advantageous for ensuring that the anti-lupus erythematosus pharmaceutical product for combined immunization according to the present invention can be administered accurately and conveniently, which is beneficial for control.

The present invention is further directed to a method of treating the anti-lupus erythematosus pharmaceutical product for combined immunization. The method comprises preparation of a walnut extract capsule, preparation of a wormwood extract capsule, preparation of an American ginseng extract capsule, and preparation of a shí hú extract capsule; and the preparation of the walnut extract capsule, the wormwood extract capsule, the American ginseng extract capsule and the shí hú extract capsule respectively comprises:

S1, screening and washing raw materials, and softening the clean raw materials by a method of cold soaking under reduced pressure;
S2, subjecting the raw materials softened in the step S1 to supercritical fluid extraction and separating to obtain an extraction liquid and a residue;

S3, pulverizing and grinding the residue obtained in step S2;

S4, spray drying the extraction liquid obtained in step S2 and adding the pulverized and ground residue obtained in step S3 thereto during the spray drying to obtain a dried raw material extract;

S5, filling a formulated amount of the raw material extract obtained in step S4 into a capsule shell to obtain a capsule.

The softening method by a method of cold soaking under reduced pressure is applied in the present invention, which rapidly introduces water by the action of negative pressure so as to accelerate the softening of the medicinal materials, ensures softening of the medicinal materials with water at normal temperature, shortens the infiltration time, reduces the loss and damage of active ingredients and decreases mildewing of medicinal materials.

The supercritical fluid extraction technology is applied in the present invention for extracting medicinal plants, in which the fluid is carbon dioxide and the extraction temperature is 30-40°C. This technology can avoid the volatilization of volatile oil and other components during the extraction process, and can maximally maintain the effective bioactive components of medicinal plants. At the same time, there is no solvent residue since the organic solvent is not applicable in the process, which ensures the natural biological activity of the raw materials, environmental protection, safety and no side effects.

The spray drying is applied in the present invention for quickly drying the extraction liquid of raw materials the invention, and the obtained product has fine and uniform particle size, good fluidity, increased specific surface area, favorable dissolution in the body, and favorable absorption by the human body. The raw material extract is spray-dried to obtain fine particles. Since the particles contain a viscous substance such as sugars, proteins or starches, the residue of the raw material is added during spray drying of the raw material extract. The residue of the raw material can adsorb the spray-dried raw material extraction liquid, and the spray-dried raw material extraction liquid has strong binding force with the residue of the raw material, which can prevent the occurrence of wall-sticking. At the same time, the raw material medicine is fully utilized, and no other extra auxiliary materials are added, which are conducive to maintain its biological activity.

The capsule prepared by the above preparation has safety and no side effects. The extraction process does not destroy the biologically active component of the original plant, and the biological active components can be fully made into an extract having a large specific surface area, which is beneficial to dissolution in the body and absorption by the human body. At the same time, it is possible to avoid the wall-sticking phenomenon or the like which occurs during the production process by using this method.

Preferably, it has been found that in S1, the method of cold soaking under reduced pressure is performed at the temperature of 10-40°C and at the pressure of -0.1 to -0.09 MPa;

in S2, the fluid for the supercritical fluid extraction is carbon dioxide, and the extraction temperature is 30-40°C;

in S4, the temperature of spray drying is 40-80°C, the drying time is 3-60 s, and the raw material extract has a particle size of 10-60 $\mu$m; and

in S5, the capsule shell is a natural plant capsule shell.

Another object of the present invention is to provide a method of preparing the anti-lupus erythematosus pharmaceutical product for combined immunization, the method comprises preparation of a walnut extract capsule, preparation of a wormwood extract capsule, preparation of an American ginseng extract capsule, and preparation of a shí hú extract capsule;

the preparation of the walnut extract capsule comprises:

S1, screening and washing walnuts, and softening the clean endodermis of walnuts by a method of cold soaking under reduced pressure; the method of cold soaking under reduced pressure is performed at the temperature of 10-40°C and at the pressure of -0.1 to -0.09 MPa;

S2, subjecting the walnuts softened in step S1 to supercritical fluid extraction and separating to obtain a walnut extraction liquid and a walnut residue; the fluid for the supercritical fluid extraction is carbon dioxide, and the extraction temperature is 30-40°C;

S3, pulverizing and grinding the walnut residue obtained in step S2;

S4, spray drying the walnut extraction liquid obtained in step S2 and adding the pulverized and ground walnut residue obtained in step S3 thereto during the spray drying to obtain a dried walnut extract; the temperature of spray drying is 40-80°C, the drying time is 3-60 s, and the walnut extract has a particle size of 10-60 $\mu$m; and

S5, filling 1 part of walnut extract obtained in step S4 into a natural plant capsule shell to obtain a walnut extract capsule;

the preparation of the wormwood extract capsule comprises:

S1, screening and washing wormwoods, and softening the clean whole herb of wormwoods by a method of cold soaking under reduced pressure; the method of cold soaking under reduced pressure is performed at the temperature of 10-40°C and at the pressure of -0.1 to -0.09 MPa;

S2, subjecting the wormwoods softened in step S 1 to supercritical fluid extraction and separating to obtain a wormwood extraction liquid and a wormwood residue; the fluid for the supercritical fluid extraction is carbon dioxide, and the extraction temperature is 30-40°C;

S3, pulverizing and grinding the wormwood residue obtained in step S2;

S4, spray drying the wormwood extraction liquid obtained in step S2 and adding the pulverized and ground wormwood residue obtained in step S3 thereto during the spray drying to obtain a dried wormwood extract; the temperature of spray drying is 40-80°C, the drying time is 3-60 s, and the wormwood extract has a particle size of 10-60 μm;

S5, filling 54 parts of wormwood extract obtained in step S4 into a natural plant capsule shell to obtain a wormwood extract capsule;

the preparation of the American ginseng extract capsule comprises:

S1, screening and washing American ginseng, and softening the clean American ginseng body by a method of cold soaking under reduced pressure; the method of cold soaking under reduced pressure is performed at the temperature of 10-40°C and at the pressure of -0.1 to -0.09 MPa;

S2, subjecting the American ginseng softened in step S1 to supercritical fluid extraction and separating to obtain an American ginseng extraction liquid and an American ginseng residue; the fluid for the supercritical fluid extraction is carbon dioxide, and the extraction temperature is 30-40°C;

S3, pulverizing and grinding the American ginseng residue obtained in step S2;

S4, spray drying the American ginseng extraction liquid obtained in step S2 and adding the pulverized and ground American ginseng residue obtained in step S3 thereto during the spray drying to obtain a dried American ginseng extract; the temperature of spray drying is 40-80°C, the drying time is 3-60 s, and the American ginseng extract has a particle size of 10-60 μm;

S5, filling 1-5 parts of the American ginseng extract obtained in step S4 into a natural plant capsule shell to obtain an American ginseng extract capsule;

the preparation of the shí hú extract capsule comprises:

S 1, screening and washing shí hú, and softening the clean stems and leaves of shí hú by a method of cold soaking under reduced pressure; the method of cold soaking under reduced pressure is performed at the temperature of 10-40°C and at the pressure of -0.1 to -0.09 MPa;

S2, subjecting the shí hú softened in step S 1 to supercritical fluid extraction and separating to obtain a shí hú extraction liquid and a shí hú residue; the fluid for the supercritical fluid extraction is carbon dioxide, and the extraction temperature is 30-40°C;

S3, pulverizing and grinding the shí hú residue obtained in step S2;

S4, spray drying the shí hú extraction liquid obtained in step S2 and adding the pulverized and ground shí hú residue obtained in step S3 thereto during the spray drying to obtain a dried shí hú extract; the temperature of spray drying is 40-80°C, the drying time is 3-60 s, and the shí hú extract has a particle size of 10-60 μm; S5, filling 1-10 parts of shí hú extract obtained in step S4 into a natural plant capsule shell to obtain a shí hú extract capsule;

the walnut is black walnut, the wormwood is Artemisia absinthium, the American ginseng is semi-wild American ginseng, and the shí hú is wild shí hú.

In the anti-lupus erythematosus pharmaceutical product for combined immunization according to the present invention, the walnut extract capsule, the wormwood extract capsule, the American ginseng extract capsule and the shí hú extract capsule are separately prepared. On the one hand, the mutual interference of the four plants can be avoided during the treatment, which is beneficial to ensuring the quality of the product. On the other hand, the administration can be varied according to the needs during the treatment, which is suitable for different types of patients.

Another object of the present invention is to provide a use of an anti-lupus erythematosus pharmaceutical product for combined immunization in the manufacture of a pharmaceutical product for preventing or treating lupus erythematosus.

Another object of the present invention is to provide a use of the anti-lupus erythematosus pharmaceutical product for combined immunization prepared by the preparation method in the manufacture of a pharmaceutical product for preventing or treating lupus erythematosus.

The following beneficial effects are obtained by using the above technical solutions:

1. Under the synergistic effects of the combination, the anti-lupus erythematosus pharmaceutical product for combined immunization of the present invention is capable of inhibiting and killing bacteria, enhancing the body immunity and quickly and effectively treating lupus erythematosus, and is also capable of further enhancing the body immunity, adjusting the in-vivo environment and repairing the original damaged immune function, restoring normal balance of the body and preventing lupus erythematosus from recurring.

2. In the preparation process of the anti-lupus erythematosus pharmaceutical product for combined immunization according to the present invention, the effective bioactive components of the medicinal plants can be extracted to the greatest extent without using organic solvents and destructive conditions, and the composition is safe and has no side effects. Meanwhile, the effective bioactive components can also be made into the particle with a large specific surface area, which is beneficial to the absorption in human body.

3. In the preparation process of the anti-lupus erythematosus pharmaceutical product for combined immunization according to the present invention, the residue of the raw materials is added during the spray drying of the raw material extract, which is beneficial to solve the wall-sticking phenomenon of the raw material extract during the production process together with the conventional anti-sticking operation, and the raw material medicine can be fully utilized to maintain its natural activity.

4. The anti-lupus erythematosus pharmaceutical product for combined immunization according to the present invention is in the form of a capsule, and thus the administration is precise and convenient, which is advantageous for preservation and stability.

**Detailed Description of the Invention**

In the examples, the walnut is selected from Black Walnut (*juglans nigra L*) from the North America. The wormwood is selected from woormwood (*Artemisia absinthium*). The American ginseng is selected from semi-wild American ginseng. The shí hú is selected from wild shí hú.

The endodermis of walnut was screened and washed, and the clean endodermis of walnut was softened by a method of cold soaking under reduced pressure at the temperature of 10-40°C and at the pressure of -0.1 to -0.09 MPa. After softening, the supercritical fluid extraction was carried out at 30-40°C using a carbon dioxide fluid, and a walnut extract and a walnut residue was obtained by separating. The walnut residue was pulverized, ground and passed through a 200-300 mesh sieve. The walnut extract was sprayed onto the sieved walnut residue by spray drying at a temperature of 40 to 80°C for a drying time of 3 to 60 s to obtain a walnut extract having a particle size of 10-60 $\mu$m. 1 part of walnut extract was filled into a natural plant capsule shell by a conventional capsule filling method to obtain a walnut extract capsule. The whole herb of wormwood was screened and washed, and the clean herb of wormwood was softened by a method of cold soaking under reduced pressure at the temperature of 10-40°C and at the pressure of -0.1 to -0.09 MPa. After softening, the supercritical fluid extraction was carried out at 30-40°C using a carbon dioxide fluid, and a wormwood extraction fluid and a wormwood residue was obtained by separating. The wormwood residue was pulverized, ground and passed through a 200-300 mesh sieve. The wormwood extraction fluid was sprayed onto the sieved wormwood residue by spray drying at a temperature of 40 to 80°C for a drying time of 3 to 60 s to obtain a wormwood extract having a particle size of 10-60 $\mu$m. The wormwood extract was filled into a natural plant capsule shell by a conventional capsule filling method to obtain a wormwood extract capsule.

The American ginseng subjected to screening and cleaning was taken, and the cleaned American ginseng body was softened by cold soaking under reduced pressure at 10-40°C and at -0.1 to -0.09 MPa. The softened American ginseng body was subjected to supercritical fluid extraction using a carbon dioxide fluid at 30-40°C, and subjected to separation to obtain the American ginseng extraction liquid and the American ginseng residue. The American ginseng residue was pulverized and ground, and passed through a 200-300-mesh sieve. The American ginseng extraction liquid was sprayed onto the sieved American ginseng residue by spray drying to obtain the American ginseng extract having the particle size of 10-60 $\mu$m, wherein the temperature of the spray drying was 40-80°C, and the drying time was 3-60 S. 1-5 parts of the American ginseng extract was taken, and filled into a natural plant capsule shell by a conventional capsule filling method to obtain the American ginseng extract capsule.

The shí hú subjected to screening and cleaning was taken, and the cleaned shí hú stems were softened by vacuum cold soaking under the conditions of 10-40°C and -0.1 to -0.09 MPa. The softened shí hú stems were subjected to supercritical fluid extraction using a carbon dioxide fluid at 30-40°C, and subjected to separation to obtain the shí hú extraction liquid and the shí hú residue. The shí hú residue was pulverized and ground, and passed through a 200-300-mesh sieve. The shí hú extraction liquid was sprayed onto the sieved shí hú residue by spray drying to obtain the shí hú extract having the particle size of 10-60 $\mu$m, wherein the temperature of the spray drying was 40-80°C, and the drying time was 3-60 S. 1-10 parts of the shí hú extract was taken, and filled into a natural plant capsule shell by a conventional capsule filling method to obtain the shí hú extract capsule.

When the anti-lupus erythematosus pharmaceutical product for combined immunization of the present invention is used, the walnut extract capsule, the wormwood extract capsule, the American ginseng extract capsule and the shí hú extract capsule are simultaneously administered in a certain ratio.

Table 1 Anti- lupus erythematosus pharmaceutical product for combined immunization of the present invention

| Extract Capsule | | Example 1 | Example 2 | Example 3 | Example 4 | Example 5 | Example 6 |
|---|---|---|---|---|---|---|---|
| Walnut Extract Capsule | Mass of walnut extract (mg) | 200 | 200 | 200 | 200 | 200 | 200 |
| | Model No.of natural plant capsule shell | 3# | 3# | 3# | 3# | 3# | 3# |
| Wormwood Extract Capsule | Mass of wormwood extract (mg) | 200 | 100 | 400 | 150 | 300 | 250 |
| | Model No.of natural plant capsule shell | 3# | 3# | 0# | 3# | 3# | 3# |
| American Ginseng Extract Capsule | Mass of American ginseng extract (mg) | 1000 | 200 | 500 | 300 | 800 | 400 |
| | Model No.of natural plant capsule shell | 0# | 3# | 0# | 3# | 0# | 0# |
| Shí hú Extract Capsule | Mass of shí húDendrobium extract (mg) | 2000 | 200 | 1000 | 500 | 1500 | 800 |
| | Model No.of natural plant capsule shell | 0# | 3# | 0# | 0# | 0# | 0# |

Comparative Example 1: Toxicology test

1. Test materials: The walnut extract, wormwood extract, American ginseng extract and shí hú extract were all prepared by Example 1. The walnut extract, wormwood extract, American ginseng extract and shí hú extract were respectively taken, treated in a 70°C water bath for 1 hour, soaked for 12 hours, and concentrated for testing.

2. Test animals:

The healthy Kunming mice provided by the Experimental Animal Center of the National Institute for the Control of Pharmaceutical and Biological Products were selected.

3. Test for micronucleated polychromatic erythrocytes in bone marrow of mice

The assay was carried out via oral gavage and the administration was performed twice at intervals of 24 hrs. 50 mice weighing 25-30 g were randomly divided into 5 groups according to body weight, 10 in each group, half male and half female. The cyclophosphamide (cp) at a dose of 50 mg/kg•bw was used as a positive control, and the distilled water was used as a negative control. The doses of the anti-lupus erythematosus pharmaceutical product for combined immunization according to the present invention were 2.00, 4.00, and 6.00 g/kg•bw, respectively, which were adjusted to the desired concentration with distilled water. The animals were sacrificed by cervical dislocation six hours after the last administration of the anti-lupus erythematosus pharmaceutical product for combined immunization according to the present invention. The bone marrow of sternum was acquired and diluted with calf serum, smeared, fixed in methanol, and stained with Giemsa. Under the light microscope, 1000 polychromatic erythrocytes (PRC) were measured per animal, and the incidence of micronucleus was determined by the permillage of micronucleus-containing PRC and statistically processed. The statistical results for the effects of the anti-lupus erythematosus pharmaceutical product for combined immunization according to the present invention on the incidence of bone marrow micronucleus in mice are shown in Table 2.
Kunming healthy mice provided by Laboratory Animal Center of National Institutes for Food and Pharmaceutical product Control were selected.

3. Mouse bone marrow polychromatic erythrocyte micronucleus test

The test was carried out by two-time intragastric administration at an interval of 24 hours. 50 mice weighing 25-30 g were randomly divided into 5 groups according to their body weight. They were randomly divided into 5 groups according to their body weight, with 10 mice in each group, half male and half female. Cyclophosphamide (cp) at a dose of 50 mg/kg•bw was used as a positive control, and distilled water was used as a negative control. The doses of the anti-lupus erythematosus pharmaceutical product for combined immunization of the present invention were 2.00, 4.00 and 6.00 g/kg•bw respectively, and were mixed with distilled water to the desired concentration. The animals were sacrificed by cervical dislocation 6 hours after the last administration of the anti-lupus erythematosus pharmaceutical product for combined immunization of the present invention. The sternum bone marrow was diluted with calf serum, fixed with methanol, and stained with Giemsa. Under the light microscope, 1000 polychromatic erythrocytes (PRC) were counted per animal, and the incidence of micronuclei was measured by the permillage of micronucleus-containing PRC and statistically processed. The results of the effects of the anti-lupus erythematosus pharmaceutical product for combined immunization of the present invention on the incidence of mouse bone marrow micronuclei are shown in Table 2.

Table 2 Results of test for micronucleated polychromatic erythrocytes in bone marrow of mice

| Gender | Dose (g/kg•bw) | Number of Animals(in Zhi) | Number of Cells Inspected(in Ge) | Number of Micronuclei (in Ge) | Permillage of Micronuclei (‰) |
|---|---|---|---|---|---|
| Male | 0.00 | 5 | 5000 | 10 | 2.0 |
| | 2.00 | 5 | 5000 | 9 | 1.8 |
| | 4.00 | 5 | 5000 | 8 | 1.6 |
| | 6.00 | 5 | 5000 | 7 | 1.4 |
| | 50mg/kg•bw (cp) | 5 | 5000 | 150 | 30.0* |
| Female | 0.00 | 5 | 5000 | 7 | 1.4 |
| | 2.00 | 5 | 5000 | 8 | 1.6 |
| | 4.00 | 5 | 5000 | 9 | 1.8 |
| | 6.00 | 5 | 5000 | 9 | 1.8 |
| | 50mg/kg•bw (cp) | 5 | 5000 | 150 | 30.0* |
| * Extremely significant difference from the negative control group ($P<0.01$). | | | | | |

It can be seen from table 2 that the micronucleus rate of each dose group of the anti-lupus erythematosus pharmaceutical product for combined immunization according to the present invention is small, and there is no significant difference between the respective dose groups and the negative control group, while the difference between the cyclophosphamide group and the negative control group is extremely significant (P<0.01). The anti-lupus erythematosus pharmaceutical product for combined immunization according to the present invention does not affect the rate of micronucleated polychromatic erythrocytes in bone marrow of mice.

4. Sperm abnormality assay in mice

50 sexually mature male mice weighing 30-35 g were randomly divided into 5 groups. The cyclophosphamide at a dose of 40 mg/kg•bw was used as a positive control, and the distilled water was used as a negative control. The doses of the anti-lupus erythematosus pharmaceutical product for combined immunization according to the present invention were 2.00, 4.00, and 6.00 g/kg•bw, respectively, and the desired concentration were adjusted with distilled water. The mice were administered via oral gavage once a day for 5 days, and the animals were sacrificed 30 days after the last gavage. The epididymis was acquired and sectioned and stained with eosin. Five animals were counted in each group and 1000 intact sperm were counted per animal. The incidence of sperm abnormality (in percentage) was calculated and statistically processed. The statistical results of the effects of the anti-lupus erythematosus pharmaceutical product for combined immunization according to the present invention on the incidence of sperm abnormality in mice are shown in Table 3.

Table 3 Statistical results of sperm abnormality assay in mice

| Dose (g/ kg•bw) | Number of Animals(in Zhi) | Number of Sperms Inspected(in Ge) | Number of Abnormal Sperms(in Ge) | Percentage of Abnormal Sperms (%) |
|---|---|---|---|---|
| 0.00 | 5 | 5000 | 81 | 1.62 |
| 2.00 | 5 | 5000 | 94 | 1.88 |
| 4.00 | 5 | 5000 | 76 | 1.52 |
| 6.00 | 5 | 5000 | 87 | 1.74 |
| 40mg/ kg•bw (cp) | 5 | 5000 | 480 | 9.6* |
| * Extremely significant difference from the negative control group (P<0.01). | | | | |

It can be seen from table 3 that the anti-lupus erythematosus pharmaceutical product for combined immunization according to the present invention does not significantly affect the incidence of sperm abnormality in the mice, and there is no significant difference between the respective dose groups and the negative control group, while the difference between the cyclophosphamide group and the negative control group is extremely significant (P<0.01). Therefore, the anti-lupus erythematosus pharmaceutical product for combined immunization according to the present invention does not affect the sperm abnormality in the mice.

The pharmaceutical products for treating lupus erythematosus and combined immunization according to the present invention as prepared in Examples 2-7 were also subjected to the same toxicological assay, and the results were consistent with that of the anti-lupus erythematosus pharmaceutical product for combined immunization according to the present invention as prepared in Example 1.

Comparative Example 2. Immunomodulatory assay

1. Test materials: The walnut extract capsule, the wormwood extract capsule, and the American ginseng extract capsule and the shí hú extract capsule were all prepared in Example 1. The recommended amount for oral administration in human is 3.4 g/day, which is calculated based on 60 kg body weight per person (one capsule for each variety), and equivalent to 0.057 g/kg•bw. The equivalent dose in the mouse is equivalent to 10 times the recommended amount for the human body, that is, 0.57 g/kg•bw (medium dose) of shaped product per day. One dose group was set for high and low doses, respectively: 1.70 g/kg•bw (high dose) of shaped product and 0.57 g/kg•bw of shaped product (low dose). The samples were formulated in sterilized water and sterilized water was used as control group. After continuous gavage for 30 days, various immune indicators were measured. Since the walnut extract capsule, the wormwood extract capsule, the American ginseng extract capsule and the shí hú extract capsule are all insoluble in water, these capsules were soaked with 10 times the recommended amount of hot water (70-80°C) for 3 times (one hour/time) and then were concentrated by evaporation on a rotary evaporator (50-60°C) to a concentration of 30 times the recommended dose, and then diluted proportionally for gavage.

2. Test animals:

75 healthy male Kunming mice weighing 18-22 g provided by the Experimental Animal Center of the National Institute for the Control of Pharmaceutical and Biological Products were selected and divided into 4 groups with 15 per group for the dose groups and 30 for the control group to carry out delayed type hypersensitivity and antibody-forming cell assays.

75 healthy male Kunming mice weighing 18-22 g provided by the Experimental Animal Center of the National Institute for the Control of Pharmaceutical and Biological Products were selected and divided into 4 groups with 15 per group for the dose groups and 30 for the control group to carry out carbon clearance assay.

3. Delayed type hypersensitivity assay (DTH) (footpad incrassation)

The mice were intraperitoneally injected with 2% (v/v, prepared with physiological saline) of SRBC (0.2 mL/mouse). Four days after sensitization, the thickness of the left and right footpads was measured, the same position was measured three times and the results were averaged. Then, 20% (v/v, prepared with physiological saline) of SRBC (20 $\mu$L/mouse) was injected subcutaneously at the measurement site, and the thickness of the left and right footpads were measured 24 hours after the injection, the same position was measured three times and the results were averaged. The degree of DTH was expressed by the difference in the thickness of the footpad before and after the injection (swelling degree of the footpads). The statistical results of the effects of the anti-lupus erythematosus pharmaceutical product for combined immunization according to the present invention on delayed type hypersensitivity (DTH) in mice are shown in Table 4.

Table 4 Results of delayed type hypersensitivity (DTH) detection

| Groups | Number of Animals (in Zhi) | Swelling Degree of Footpad (mm) |
| --- | --- | --- |
| Control | 30 | 0.573±0.109 |
| Low Dose | 15 | 0.663±0.106 |
| Medium Dose | 15 | 0.712±0.105 |
| High Dose | 15 | 0.700±0.102 |

The mice were orally administered with different doses of the anti-lupus erythematosus pharmaceutical product for combined immunization according to the present invention for 30 days, and the results were statistically processed. As seen from Table 4, as for the swelling degree of the footpad, there was an extremely significant difference between the low, medium and high dose groups and the control group ($P<0.01$). In other words, the anti-lupus erythematosus pharmaceutical product for combined immunization according to the present invention can significantly enhance the delayed type hypersensitivity in mice.

4. Antibody-forming cell assay (modified Jerne plaque assay)

Mice were immunized by intraperitoneal injection of 0.2 mL of 2% (v/v, prepared with physiological saline) of SRBC (0.2 mL per mouse). After 5 days, the mice were sacrificed, and the spleen was removed, shredded, and prepared as a cell suspension using Hank's solution. The cell suspension was washed, centrifuged twice, and suspended in 5 mL of Hank's solution. After heating and dissolving the surface medium (agarose), it was mixed with an equal amount of double Hank's solution and dispensed into small tubes with 0.5 ml per tube. 10% (v/v, prepared with SA solution) of SRBC 50 $\mu$L and 20 $\mu$L spleen cell suspension were added to the tubes. After quickly mixing, the mixture was poured onto a slide coated with agarose. After the agar solidified, the slide was placed horizontally on the slide holder and incubated in a carbon dioxide incubator for 1.5 hr. The complement diluted with SA buffer (1:10) was added to the groove of the slide holder. After incubation for 1.5 hr, the number of hemolysis plaques was counted. The statistical results of the effects of the anti-lupus erythematosus pharmaceutical product for combined immunization according to the present invention on the number of antibody-forming cells are shown in Table 5.

Table 5 Results of antibody-forming cell detection

| Groups | Number of Animals (in Zhi) | Number of Hemolytic Plaques (x10³/a whole spleen) |
|---|---|---|
| Control | 22 | 115.9±50.5 |
| Low Dose | 11 | 108.2±43.6 |
| Medium Dose | 13 | 141.6±51.3 |
| High Dose | 12 | 129.6±101.3 |

The mice were orally administered with different doses of the anti-lupus erythematosus pharmaceutical product for combined immunization according to the present invention for 30 days, and the results were statistically processed. As seen from Table 5, as for the swelling degree of the footpad, there was no extremely significant difference between the low, medium and high dose groups and the control group (P>0.05). In other words, the anti-lupus erythematosus pharmaceutical product for combined immunization according to the present invention does not affect the number of antibody-forming cells in mice.

5. Carbon clearance assay in mice

The tail vein of the mice was injected with Indian ink diluted with physiological saline (1:4) at a dose of 0.1 mL/10 g body weight, and timing was started immediately after the injection. 20 $\mu$L of blood was taken from the angular vein 2 and 10 minutes after the injection, added into 2 mL of 0.1% $Na_2CO_3$ solution, and shaken well. The 0.1% $Na_2CO_3$ solution was used as a blank control, and the optical density value (OD) was measured at a wavelength of 600 nm using a 721 spectrophotometer. The mice were sacrificed, the liver and spleen were removed and weighed (body weight is expressed in m, liver weight is expressed in $m_1$, spleen weight is expressed in $m_2$), and the phagocytic index a was calculated as follows:

$$K = \frac{\lg OD_1 - \lg OD_2}{t_2 - t_1} \qquad \text{(Formula 1-1)}$$

$$a = \frac{m}{m_2 + m_1} \times \sqrt[3]{k} \qquad \text{(Formula 1-2)}$$

The statistical results of the effects of the anti-lupus erythematosus pharmaceutical product for combined immunization according to the present invention on the monocyte-macrophage phagocytosis in mice are shown in Table 6.

Table 6 Results of mouse carbon clearance test

| Groups | Number of Animals (in Zhi) | Phagocytic Index |
|---|---|---|
| Control | 25 | 6.01±0.52 |
| Low Dose | 15 | 6.33±0.40 |
| Medium Dose | 14 | 5.89±0.48 |
| High Dose | 14 | 5.77±0.43 |

The mice were orally administered with different doses of the anti-lupus erythematosus pharmaceutical product for combined immunization according to the present invention for 30 days, and the results were statistically processed. As seen from Table 6, as for the swelling degree of the footpad, there was no extremely significant difference between the low, medium and high dose groups and the control group (P>0.05). In other words, the anti-lupus erythematosus pharmaceutical product for combined immunization according to the present invention does not affect the monocyte-macrophage carbon clearance in mice.
The mice were orally administered with different doses of the anti-lupus erythematosus pharmaceutical product for combined immunization according to the present invention for 30 days. As seen from Tables 4-6, the anti-lupus erythematosus pharmaceutical product for combined immunization according to the present invention can significantly enhance the delayed type hypersensitivity in mice, does not affect the number of antibody-forming cells and the monocyte-macrophage carbon clearance in mice. Therefore, the anti-lupus erythematosus pharmaceutical product for combined immunization according to the present invention has an effect of enhancing cellular immune function.

The pharmaceutical products for treating lupus erythematosus and combined immunization according to the present invention as prepared in Examples 2-7 were also subjected to the same immunomodulatory assay, and the results were consistent with that of the anti-lupus erythematosus pharmaceutical product for combined immunization according to the present invention as prepared in Example 1.

Comparative Example 3. Hygienic inspection of preparations

With reference to GB4789-94 and GB4789.15-94, the anti-lupus erythematosus pharmaceutical product for combined immunization according to the present invention as prepared in Example 1 was tested for coliform bacteria, total number of colonies, pathogenic bacteria, mold, and yeast count. A set of samples just produced and a set of samples placed at 25°C/60% RH for 3 years were tested separately, and both sets of samples were samples from the same production batch. Ten samples were taken in parallel for each set of samples. The hygienic inspection results are shown in Table 7.

Table 7 Statistical results of hygienic inspection of preparations

| Capsule / Detection Item | Walnut Extract Capsule | | Wormwood Extract Capsule | | American Ginseng Extract Capsule | | shí hú Extract Capsule | |
|---|---|---|---|---|---|---|---|---|
| | 0 Day | 3 Years | 0 Day | 3 Years | 0 Day | 3 Years | 0 Day | 3 Years |
| Coliform Bacteria (MPN/100g) | <30 | <30 | <30 | <30 | <30 | <30 | <30 | <30 |
| Total Number of Colonies (CFU/g) | <10 | <10 | <10 | <10 | <10 | <10 | <10 | <10 |
| Staphylococcus Aureus | Not Detected | Not Detected | Not Detected | Not Detected | Not Detected | Not Detected | Not Detected | Not Detected |
| Haemolytic Streptococcus | Not Detected | Not Detected | Not Detected | Not Detected | Not Detected | Not Detected | Not Detected | Not Detected |
| Shigella | Not Detected | Not Detected | Not Detected | Not Detected | Not Detected | Not Detected | Not Detected | Not Detected |
| Salmonella | Not Detected | Not Detected | Not Detected | Not Detected | Not Detected | Not Detected | Not Detected | Not Detected |
| Mold(CFU/g) | Not Detected | Not Detected | Not Detected | Not Detected | Not Detected | Not Detected | Not Detected | Not Detected |
| Yeast(CFU/g) | Not Detected | Not Detected | Not Detected | Not Detected | Not Detected | Not Detected | Not Detected | Not Detected |

As can be seen from Table 7, the anti-lupus erythematosus pharmaceutical product for combined immunization placed at 25°C/60% RH for 3 years after production passed the hygienic inspection.

Similar hygienic inspection was also performed on the anti-lupus erythematosus pharmaceutical product for combined immunization of the present invention prepared in Example 2 to Example 7, and the results were the same as those of the anti-lupus erythematosus pharmaceutical product for combined immunization of the present invention prepared in Example 1.

Comparative Example 4: Clinical trials

Clinical trials were performed in patient cases with lupus erythematosus. The inclusion criteria were: pathologically or cytologically confirmed diagnosis. Among them, anyone who satistied 4 or more of the following 11 criteria can be diagnosed as having systemic lupus erythematosus: (1) butterfly erythema on the cheek; (2) discoid erythema; (3) solar; (4) oral ulcer; (5) non-aggressive; (6) pleurisy, pericarditis; (7) proteinuria, more than 0.5 gram per day; (8) abnormalities of nervous system: convulsions or mental illness; (9) hematological abnormalities, hemolytic, or less than $4\times10^9$/L of white blood cells, or less than $1.5\times10^9$/L of lymphocytes, or less than $100\times10^9$/L of platelets; (10) Immunological abnormalities, positive lupus cell, positive anti-double-stranded-dna antibody or positive anti-sm antibody, or false positive serum test; and (11) positive immunofluorescent anti-nuclear antibody. The examination means comprise blood routine examination (including hemoglobins, red blood cells, white blood cells and platelets), urine routine examination and blood biochemical examination.

A total of 200 patients with systemic lupus erythematosus were enrolled, including 30 males and 170 females, aged 20-60 years, with an average age of 30 years.

Therapeutic method: The whole group of patients were orally administered with the anti-lupus erythematosus pharmaceutical product for combined immunization of the present invention prepared in Example 1, one capsule each time for each of the capsules, twice a day (once in the morning and once in the evening), for one treatment course of six months.

After treatment, there were 25 patients who were completely cured within 2 months, 44 patients who were completely cured within 2-4 months, and 61 patients who were completely cured within 4-6 months. After the administartion for one treatment course, 106 of the 130 patients who had been cured did not relapse within 2 years, while the remaining 24 patients had relieved symptoms than before in relapses. By further taking the anti-lupus erythematosus pharmaceutical product for combined immunization of the present invention for one treatment course, all of these 24 patients suffering from the relapses were completely cured within 2 months.

Toxic and side reaction observation: No adverse reactions such as heart, liver and kidney function were observed in the whole patients during the treatment period, and no abnormalities were found in the peripheral blood. No allergic phenomena such as rash were observed.

The above description is only a preferred embodiment of the present invention, and the scope of protection of the present invention is not limited to the above embodiments. All the technical solutions under the inventive concept of the invention fall within the protection scope of the present invention. It should be noted that various modifications and refinements made by those skilled in the art without departing from the principles of the invention are also considered to be within the protection scope of the invention.

**Claims**

1. An anti-lupus erythematosus pharmaceutical product for combined immunization, **characterized by** comprising an extract of a walnut, an extract of a wormwood, an extract of an American ginseng and an extract of a shí hú as raw materials; the extract of a walnut, the extract of a wormwood, the extract of an American ginseng and the extract of a shí huare in a weight ratio of 1:(0.5-2):(1-5):(1-10).

2. The anti-lupus erythematosus pharmaceutical product for combined immunization according to claim 1, **characterized in that** the walnut is black walnut, the wormwood is Artemisia absinthium, the American ginseng is semi-wild American ginseng, and the shí hú is wild shí hú.

3. The anti-lupus erythematosus pharmaceutical product for combined immunization according to claim 1, **characterized in that** the weight ratio of the extract of a walnut to the extract of a wormwood to the extract of an American ginseng to the extract of a shí hú is 1:1:5:10.

4. The anti-lupus erythematosus pharmaceutical product for combined immunization according to claim 1 or 2 or 3, **characterized in that** a formulation of the pharmaceutical product is a capsule.

5. A preparation method of the anti-lupus erythematosus pharmaceutical product for combined immunization according to claim 4, **characterized in that** the method comprises preparation of a walnut extract capsule, preparation of a wormwood extract capsule, preparation of an American ginseng extract capsule, and preparation of a shí hú extract capsule; the preparation of the walnut extract capsule, the prepration of the wormwood extract capsule, the preparation of the American ginseng extract capsule, and the preparation of the shí hú extract capsule comprise:

S1, screening and washing raw materials, and softening the clean raw materials by a method of cold soaking under reduced pressure;

S2, subjecting the raw materials softened in step S1 to supercritical fluid extraction and separating to obtain an extraction liquid and a residue;

S3, pulverizing and grinding the residue obtained in step S2;

S4, spray drying the extraction liquid obtained in step S2 and adding the pulverized and ground residue obtained in step S3 thereto during the spray drying to obtain a dried raw material extract;

S5, filling a formulated amount of the raw material extract obtained in step S4 into a capsule shell to obtain a capsule.

6. The preparation method of the anti-lupus erythematosus pharmaceutical product for combined immunization according to claim 5, **characterized in that**:

in S1, the method of cold soaking under reduced pressure is performed at the temperature of 10-40°C and at the pressure of -0.1 to -0.09 MPa;

in S2, the fluid for the supercritical fluid extraction is carbon dioxide, and the extraction temperature is 30-40°C;

in S4, the temperature of spray drying is 40-80°C, the drying time is 3-60 s, and the raw material extract has a particle size of 10-60 $\mu$m;

in S5, the capsule shell is a natural plant capsule shell.

7. The preparation method of the anti-lupus erythematosus pharmaceutical product for combined immunization according to claim 2, **characterized in that**:

the method comprises the preparation of a walnut extract capsule, the preparation of a wormwood extract capsule, the preparation of an American ginseng extract capsule, and the preparation of a shí hú extract capsule; the preparation of the walnut extract capsule comprises:

S1, screening and washing walnuts, and softening the clean endodermis of walnuts by a method of cold soaking under reduced pressure; the method of cold soaking under reduced pressure is performed at the temperature of 10-40°C and at the pressure of -0.1 to -0.09 MPa;

S2, subjecting the walnuts softened in step S1 to supercritical fluid extraction and separating to obtain a walnut extraction liquid and a walnut residue; the fluid for the supercritical fluid extraction is carbon dioxide, and the extraction temperature is 30-40°C;

S3, pulverizing and grinding the walnut residue obtained in step S2;

S4, spray drying the walnut extraction liquid obtained in step S2 and adding the pulverized and ground walnut residue obtained in step S3 thereto during the spray drying to obtain a dried walnut extract; the temperature of spray drying is 40-80°C, the drying time is 3-60 s, and the walnut extract has a particle size of 10-60 $\mu$m;

S5, filling 1 part of the walnut extract obtained in step S4 into a natural plant capsule shell to obtain a walnut extract capsule;

the preparation of the wormwood extract capsule comprises:

S1, screening and washing wormwoods, and softening the clean whole herb of wormwoods by a method of cold soaking under reduced pressure; the method of cold soaking under reduced pressure is performed at the temperature of 10-40°C and at the pressure of -0.1 to -0.09 MPa;

S2, subjecting the wormwoods softened in step S1 to supercritical fluid extraction and separating to obtain a wormwood extraction liquid and a wormwood residue; the fluid for the supercritical fluid extraction is carbon dioxide, and the extraction temperature is 30-40°C;

S3, pulverizing and grinding the wormwood residue obtained in step S2;

S4, spray drying the wormwood extraction liquid obtained in step S2 and adding the pulverized and ground wormwood residue obtained in step S3 thereto during the spray drying to obtain a dried wormwood extract; the temperature of spray drying is 40-80°C, the drying time is 3-60 s, and the wormwood extract has a particle size of 10-60 μm;
S5, filling 54 parts of the wormwood extract obtained in step S4 into a natural plant capsule shell to obtain a wormwood extract capsule;

the preparation of the American ginseng extract capsule comprises:

S1, screening and washing American ginseng, and softening the clean American ginseng body by a method of cold soaking under reduced pressure; the method of cold soaking under reduced pressure is performed at the temperature of 10-40°C and at the pressure of -0.1 to -0.09 MPa;
S2, subjecting the American ginseng softened in step S1 to supercritical fluid extraction and separating to obtain an American ginseng extraction liquid and an American ginseng residue; the fluid for the supercritical fluid extraction is carbon dioxide, and the extraction temperature is 30-40°C;
S3, pulverizing and grinding the American ginseng residue obtained in step S2;
S4, spray drying the American ginseng extraction liquid obtained in step S2 and adding the pulverized and ground American ginseng residue obtained in step S3 thereto during the spray drying to obtain a dried American ginseng extract; the temperature of spray drying is 40-80°C, the drying time is 3-60 s, and the American ginseng extract has a particle size of 10-60 μm;
S5, filling 1-5 parts of American ginseng extract obtained in step S4 into a natural plant capsule shell to obtain an American ginseng extract capsule;

the preparation of the shí hú extract capsule comprises:

S1, screening and washing shí hú, and softening the clean stems and leaves of shí hú by a method of cold soaking under reduced pressure; the method of cold soaking under reduced pressure is performed at the temperature of 10-40°C and at the pressure of -0.1 to -0.09 MPa;
S2, subjecting the shí hú softened in step S1 to supercritical fluid extraction and separating to obtain a shí hú extraction liquid and a shí hú residue; the fluid for the supercritical fluid extraction is carbon dioxide, and the extraction temperature is 30-40°C;
S3, pulverizing and grinding the shí hú residue obtained in step S2;
S4, spray drying the shí hú extraction liquid obtained in step S2 and adding the pulverized and ground shí hú residue obtained in step S3 thereto during the spray drying to obtain a dried shí hú extract; the temperature of spray drying is 40-80°C, the drying time is 3-60 s, and the shí hú extract has a particle size of 10-60 μm;
S5, filling 1-5 parts of the shí hú extract obtained in step S4 into a natural plant capsule shell to obtain shí hú extract capsule;

the walnut is black walnut, the wormwood is Artemisia absinthium, the American ginseng is semi-wild American ginseng, and the shí hú is wild shí hú.

8. Use of the anti-lupus erythematosus pharmaceutical product for combined immunization according to claim 1 in preparation of pharmaceutical products for preventing or treating lupus erythematosus.

9. Use of the anti-lupus erythematosus pharmaceutical product for combined immunization prepared by the preparation method according to any of claims 5-7 in preparation of pharmaceutical products for preventing or treating lupus erythematosus.

# INTERNATIONAL SEARCH REPORT

| International application No. |
|---|
| **PCT/CN2016/078649** |

**A. CLASSIFICATION OF SUBJECT MATTER**

A61K 36/8984 (2006.01) i; A61K 36/52 (2006.01) i; A61K 36/282 (2006.01) i; A61K 36/258 (2006.01) i; A61K 9/48 (2006.01) i; A61P 37/02 (2006.01) i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CPRSABS, CNABS, CNMED, TWABS, MOABS, HKABS, TWMED, CNTXT, TWTXT, CJFD, MEDNPL, CSCD, SIPONPL, DWPI, SIPOABS, CPEA, USTXT, WOTXT, EPTXT, CATXT, JPTXT, CNPAT, Chinese Pharmaceutical Patent Database and Retrieval System, WPI, EPODOC, CNKI: mandshurica Maxim, Artemisia carvifolia, Dendrobium candidum, American ginseng, juglans+, walnut, artemisia+, pan ax quinquefolium, dendrobi+, supercritical fluid extraction, SFE, spray drying, lupus erythematosus, LE, SLE, DLE, SCIE, LEP, NLE, DIL

**C. DOCUMENTS CONSIDERED TO BE RELEVANT**

| Category* | Citation of document, with indication, where appropriate, of the relevant passages | Relevant to claim No. |
|---|---|---|
| E | CN 105770474 A (BEIJING LUDEKAIQI BUSINESS CO., LTD.), 20 July 2016 (20.07.2016), claims 1-9 | 1-9 |
| Y | LI, Qing; "Research and Application of Artemisia Annua", CHINA MEDICAL HERALD, vol. 5, no. 36, 31 December 2008 (31.12.2008), page 25, column 2 | 1-9 |
| Y | US 2003068358 A1 (BOGAR, A.G.), 10 April 2003 (10.04.2003), description, paragraphs 12 and 23 | 1-9 |
| Y | YANG, Mingjing et al., "Effects of Dendrobium Candicum Capsules On Immune Responses In Mouse", JIANGSU JOURNAL OF PREVENTIVE MEDICINE, vol. 19, no. 04, 31 December 2008 (31.12.2008), pages 11-13 | 1-9 |
| Y | CN 105169105 A (NINGBO YUFANGTANG BIOTECHNOLOGY CO., LTD.), 23 December 2015 (23.12.2015), claims 3 and 4 | 5-7 |

☒ Further documents are listed in the continuation of Box C.    ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 December 2016 (02.12.2016) | **11 January 2017 (11.01.2017)** |
| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHANG, Zhonghui** Telephone No.: (86-10) **62089324** |

Form PCT/ISA/210 (second sheet) (July 2009)

## INTERNATIONAL SEARCH REPORT

| International application No. |
| --- |
| **PCT/CN2016/078649** |

**C (Continuation).** **DOCUMENTS CONSIDERED TO BE RELEVANT**

| Category* | Citation of document, with indication, where appropriate, of the relevant passages | Relevant to claim No. |
| --- | --- | --- |
| A | LI, Weiyang et al., "Syndrome Differentiation Depending Treatment of Chinese Medicine of Systemic Lupus Erythematosus", SHAANXI JOURNAL OF TRADITIONAL CHINESE MEDICINE, vol. 23, no. 11, 30 November 2002 (30.11.2002), pages 1056 and 1057 | 1-9 |

Form PCT/ISA/210 (continuation of second sheet) (July 2009)

## INTERNATIONAL SEARCH REPORT
Information on patent family members

| | International application No. |
|---|---|
| | **PCT/CN2016/078649** |

| Patent Documents referred in the Report | Publication Date | Patent Family | Publication Date |
|---|---|---|---|
| CN 105770474 A | 20 July 2016 | None | |
| US 2003068358 A1 | 10 April 2003 | US 7135198 B2 | 14 November 2006 |
| | | EP 1297751 A1 | 02 April 2003 |
| CN 105169105 A | 23 December 2015 | None | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 478994 A

- GB 47891594 A

**Non-patent literature cited in the description**

- **SONG XIAOPING et al.** proposed Chinese medicine and Western medicine therapies of cutaneous lupus erythematosus in Progress and Thinking on the Treatment of Cutaneous Lupus Erythematosus with Chinese Medicine and Western Medicine. *Hainan Medicine,* 2010, vol. 21 (21